(19) Europäisches Patentamt / European Patent Office / Office européen des brevets

(11) **EP 4 358 837 B1**

(12) **EUROPEAN PATENT SPECIFICATION**

(45) Date of publication and mention
of the grant of the patent:
**26.11.2025  Bulletin 2025/48**

(21) Application number: **22878848.5**

(22) Date of filing: **04.10.2022**

(51) International Patent Classification (IPC):
**A61B 5/08** *(2006.01)*          **A61B 5/05** *(2021.01)*
**A61B 5/113** *(2006.01)*

(52) Cooperative Patent Classification (CPC):
**A61B 5/0816; A61B 5/0507;** A61B 5/002;
A61B 5/7225; A61B 5/7257; H04W 84/12

(86) International application number:
**PCT/KR2022/014901**

(87) International publication number:
**WO 2023/059027 (13.04.2023 Gazette 2023/15)**

(54) **METHODS FOR RESPIRATION RATE DETECTION WITH WI-FI**

VERFAHREN ZUR ATMUNGSRATENDETEKTION MIT WI-FI

PROCÉDÉS DE DÉTECTION DE FRÉQUENCE DE RESPIRATION AVEC WI-FI

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.10.2021  US 202163251974 P
28.09.2022  US 202217936357**

(43) Date of publication of application:
**01.05.2024  Bulletin 2024/18**

(73) Proprietor: **Samsung Electronics Co., Ltd.
Suwon-si, Gyeonggi-do 16677 (KR)**

(72) Inventors:
• **CHANG, Hao Hsuan**
  **Suwon-si, 16677 (KR)**
• **CHEN, Hao**
  **Suwon-si, 16677 (KR)**
• **RATNAM, Vishnu**
  **Suwon-si, 16677 (KR)**
• **SEHGAL, Abhishek**
  **Suwon-si, 16677 (KR)**
• **ZHANG, Jianzhong**
  **Suwon-si, 16677 (KR)**

(74) Representative: **HGF
HGF Limited
1 City Walk
Leeds LS11 9DX (GB)**

(56) References cited:
**WO-A1-2017/156492          WO-A1-2017/156492
WO-A1-86/05965          CN-B- 110 013 252
US-A1- 2016 022 145          US-A1- 2021 186 369
US-B2- 10 735 298**

• **WANG XUYU ET AL: "PhaseBeat: Exploiting CSI
Phase Data for Vital Sign Monitoring with
Commodity WiFi Devices", PROCEEDINGS OF
THE INTERNATIONAL CONFERENCE ON
DISTRIBUTED COMPUTING SYSTEMS, IEEE
COMPUTER SOCIETY, US, 5 June 2017
(2017-06-05), pages 1230 - 1239, XP033123076,
ISSN: 1063-6927, [retrieved on 20170713], DOI:
10.1109/ICDCS.2017.206**
• **ZHANG FUSANG ET AL: "Unlocking the
Beamforming Potential of LoRa for Long-range
Multi-target Respiration Sensing",
PROCEEDINGS OF THE ACM ON INTERACTIVE,
MOBILE, WEARABLE AND UBIQUITOUS
TECHNOLOGIES, ACMPUB27, NEW YORK, NY,
USA, vol. 5, no. 2, 24 June 2021 (2021-06-24),
pages 1 - 25, XP059004804, DOI: 10.1145/3463526**

EP 4 358 837 B1

## Description

### Technical Field

The present disclosure relates generally to wireless communication systems and, more specifically, the present disclosure relates to a system and method for respiration rate detection using Wi-Fi.

### Background Art

Respiration rate detection plays an important role in various applications of health care, such as sleep apnea detection and early detection of chronic respiratory diseases.
The paper "PhaseBeat: Exploiting CSI Phase Data for Vital Sign Monitoring with Commodity WiFi Devices" by Wang Xuyu et al, Proceedings of the International Conference on Distributed Computing Systems, 5 June 2017, pages 1230-1239, discusses CSI phase difference data. WO 2017/156492 A1 discusses vital sign detection and monitoring based on CSI.
The paper "Unlocking the Beamforming Potential of LoRa for Long-range Multi-target Respiration Sensing" by Zhang Fusang et al, Proceedings of the ACM on Interactive, Mobile, Wearable and Ubiquitous Technologies, vol. 5, no. 2, 24 June 2021, pages 1-25 discusses multi-target LoRa sensing. US 10735298 B2 discusses methods for detecting an monitoring vital signs.

### Disclosure of Invention

### Technical Problem

Conventional respiration rate detection techniques require patients to wear devices such as nasal tubes, capnometers, or chest pressure sensors, which are expensive and uncomfortable to wear. To enable contact-free respiration rate detection, recently respiration monitoring with Doppler radar, ultra-wide band (UWB) radar and frequency-modulated continuous wave (FMCW) radar have been proposed. Although these radar systems allow relatively precise measurements, they are very expensive due to the cost of dedicated hardware.

### Solution to Problem

The present disclosure relates to a system and method for respiration rate detection using Wi-Fi. The invention is defined in the independent claims.
Other technical features may be readily apparent to one skilled in the art from the following figures, descriptions, and claims.

### Advantageous Effects of Invention

Before undertaking the DETAILED DESCRIPTION below, it may be advantageous to set forth definitions of certain words and phrases used throughout this patent document. The term "couple" and its derivatives refer to any direct or indirect communication between two or more elements, whether or not those elements are in physical contact with one another. The terms "transmit," "receive," and "communicate," as well as derivatives thereof, encompass both direct and indirect communication. The terms "include" and "comprise," as well as derivatives thereof, mean inclusion without limitation. The term "or" is inclusive, meaning and/or. The phrase "associated with," as well as derivatives thereof, means to include, be included within, interconnect with, contain, be contained within, connect to or with, couple to or with, be communicable with, cooperate with, interleave, juxtapose, be proximate to, be bound to or with, have, have a property of, have a relationship to or with, or the like. The term "controller" means any device, system, or part thereof that controls at least one operation. Such a controller may be implemented in hardware or a combination of hardware and software and/or firmware. The functionality associated with any particular controller may be centralized or distributed, whether locally or remotely. The phrase "at least one of," when used with a list of items, means that different combinations of one or more of the listed items may be used, and only one item in the list may be needed. For example, "at least one of: A, B, and C" includes any of the following combinations: A, B, C, A and B, A and C, B and C, and A and B and C.
Moreover, various functions described below can be implemented or supported by one or more computer programs, each of which is formed from computer readable program code and embodied in a computer readable medium. The terms "application" and "program" refer to one or more computer programs, software components, sets of instructions, procedures, functions, objects, classes, instances, related data, or a portion thereof adapted for implementation in a suitable computer readable program code. The phrase "computer readable program code" includes any type of computer code, including source code, object code, and executable code. The phrase "computer readable medium" includes any type of medium capable of being accessed by a computer, such as read only memory (ROM), random access memory (RAM), a hard disk drive, a compact disc (CD), a digital video disc (DVD), or any other type of memory. A "non-transitory" computer readable medium excludes wired, wireless, optical, or other communication links that transport transitory electrical or other signals. A non-transitory computer readable medium includes media where data can be permanently stored and media where data can be stored and later overwritten, such as a rewritable optical disc or an erasable memory device.

Definitions for other certain words and phrases are provided throughout this patent document. Those of ordinary skill in the art should understand that in many if not most instances, such definitions apply to prior as well as future uses of such defined words and phrases.

## Brief Description of Drawings

For a more complete understanding of the present disclosure and its advantages, reference is now made to the following description taken in conjunction with the accompanying drawings, in which like reference numerals represent like parts:

FIGURE 1 illustrates an example wireless network according to embodiments of the present disclosure;
FIGURE 2A illustrates an example AP according to embodiments of the present disclosure;
FIGURE 2B illustrates an example STA according to embodiments of this disclosure;
FIGURE 3 illustrates an example process for respiration rate detection using Wi-Fi according to embodiments of the present disclosure;
FIGURE 4 illustrates an example anomaly removal operation for the process of FIGURE 3, according to embodiments of the present disclosure;
FIGURE 5 illustrates an example CSI compensation operation for the process of FIGURE 3, according to embodiments of the present disclosure;
FIGURE 6 illustrates an example phase compensation operation for the process of FIGURE 3, according to embodiments of the present disclosure;
FIGURE 7 illustrates an example amplitude compensation operation for the process of FIGURE 3, according to embodiments of the present disclosure;
FIGURE 8 illustrates an example multi-person respiration rate detection operation for the process of FIGURE 3, according to embodiments of the present disclosure;
FIGURE 9 illustrates RER calculation for the multi-person respiration rate detection operation of FIGURE 8, according to embodiments of the present disclosure;
FIGURE 10 illustrates a chart showing FFT of one subcarrier, according to embodiments of the present disclosure;
FIGURE 11 illustrates a FFT peak detection operation for the multi-person respiration rate detection operation of FIGURE 8, according to embodiments of the present disclosure;
FIGURES 12A and 12B illustrate charts showing CSI trajectories of different shapes;
FIGURE 13 illustrates a method of counting people with the same respiration rate according to embodiments of the present disclosure;
FIGURE 14 illustrates an example respiration rate matching operation for the multi-person respiration rate detection operation of FIGURE 8, according to embodiments of the present disclosure; and
FIGURE 15 illustrates a flow chart of a method for respiration rate detection using Wi-Fi according to embodiments of the present disclosure.

## Mode for the Invention

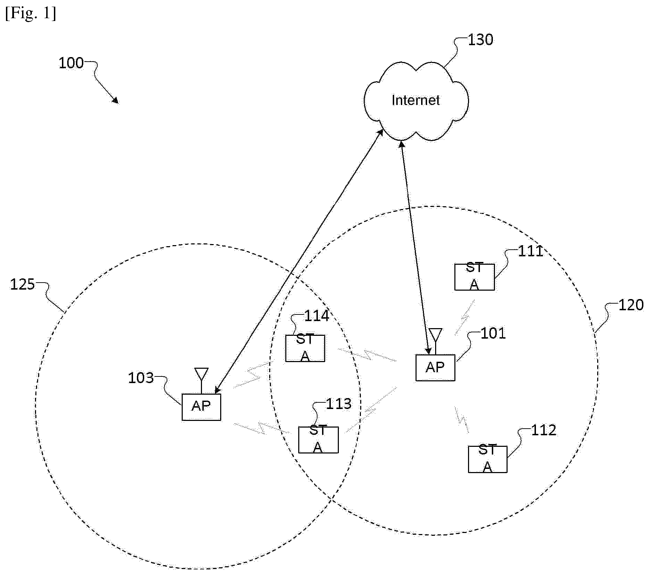

FIGURES 1 through 15, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged system or device.

Aspects, features, and advantages of the disclosure are readily apparent from the following detailed description, simply by illustrating a number of particular embodiments and implementations, including the best mode contemplated for carrying out the disclosure. The disclosure is also capable of other and different embodiments, and its several details can be modified in various obvious respects. Accordingly, the drawings and description are to be regarded as illustrative in nature, and not as restrictive. The disclosure is illustrated by way of example, and not by way of limitation, in the figures of the accompanying drawings.

The present disclosure covers several components which can be used in conjunction or in combination with one another or can operate as standalone schemes. Certain embodiments of the disclosure may be derived by utilizing a combination of several of the embodiments listed below. Also, it should be noted that further embodiments may be derived by utilizing a particular subset of operational steps as disclosed in each of these embodiments. This disclosure should be understood to cover all such embodiments.

FIGURE 1 illustrates an example wireless network 100 according to various embodiments of the present disclosure. The embodiment of the wireless network 100 shown in FIGURE 1 is for illustration only. Other embodiments of the wireless network 100 could be used without departing from the scope of this disclosure.

The wireless network 100 includes access points (APs) 101 and 103. The APs 101 and 103 communicate with at least one network 130, such as the Internet, a proprietary Internet Protocol (IP) network, or other data network. The AP 101 provides wireless access to the network 130 for a plurality of stations (STAs) 111-114 within a coverage area 120 of the AP 101. The APs 101-103 may communicate with each other and with the STAs 111-114 using Wi-Fi or other WLAN communication techniques.

Depending on the network type, other well-known terms may be used instead of "access point" or "AP," such as "router" or "gateway." For the sake of convenience, the term "AP" is used in this disclosure to refer to network infrastructure components that provide wireless access to remote terminals. In WLAN, given that the AP also contends for the wireless channel, the AP may also be referred to as a STA. Also, depending on the network type, other well-known terms may be used instead of "station" or "STA," such as "mobile station," "subscriber station," "remote terminal," "user equipment," "wireless terminal," or "user device." For the sake of convenience, the terms "station" and "STA" are used in this disclosure to refer to remote wireless equipment that wirelessly accesses an AP or contends for a wireless channel in a WLAN, whether the STA is a mobile device (such as a mobile telephone or smartphone) or is normally considered a stationary device (such as a desktop computer, AP, media player, stationary sensor, television, etc.).

Dotted lines show the approximate extents of the coverage areas 120 and 125, which are shown as approximately circular for the purposes of illustration and explanation only. It should be clearly understood that the coverage areas associated with APs, such as the coverage areas 120 and 125, may have other shapes, including irregular shapes, depending upon the configuration of the APs and variations in the radio environment associated with natural and man-made obstructions.

As described in more detail below, one or more of the APs may include circuitry and/or programming for respiration rate detection using Wi-Fi. Although FIGURE 1 illustrates one example of a wireless network 100, various changes may be made to FIGURE 1. For example, the wireless network 100 could include any number of APs and any number of STAs in any suitable arrangement. Also, the AP 101 could communicate directly with any number of TAs and provide those STAs with wireless broadband access to the network 130. Similarly, each AP 101-103 could communicate directly with the network 130 and provide STAs with direct wireless broadband access to the network 130. Further, the APs 101 and/or 103 could provide access to other or additional external networks, such as external telephone networks or other types of data networks.

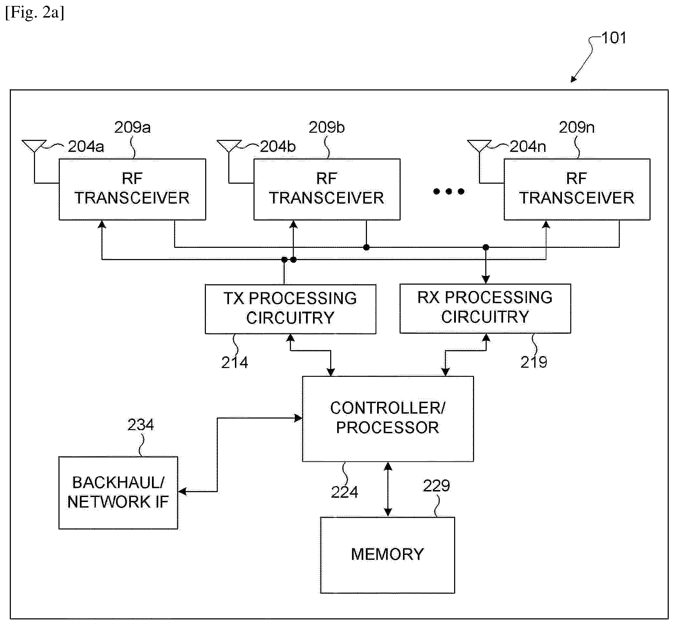

FIGURE 2A illustrates an example AP 101 according to embodiments of the present disclosure. The embodiment of the AP 101 illustrated in FIGURE 2A is for illustration only, and the AP 103 of FIGURE 1 could have the same or similar configuration. However, APs come in a wide variety of configurations, and FIGURE 2A does not limit the scope of this disclosure to any particular implementation of an AP.

The AP 101 includes multiple antennas 204a-204n, multiple RF transceivers 209a-209n, transmit (TX) processing circuitry 214, and receive (RX) processing circuitry 219. The AP 101 also includes a controller/processor 224, a memory 229, and a backhaul or network interface 234. The RF transceivers 209a-209n receive, from the antennas 204a-204n, incoming RF signals, such as signals transmitted by STAs in the network 100. The RF transceivers 209a-209n down-convert the incoming RF signals to generate IF or baseband signals. The IF or baseband signals are sent to the RX processing circuitry 219, which generates processed baseband signals by filtering, decoding, and/or digitizing the baseband or IF signals. The RX processing circuitry 219 transmits the processed baseband signals to the controller/-processor 224 for further processing.

The TX processing circuitry 214 receives analog or digital data (such as voice data, web data, e-mail, or interactive video game data) from the controller/processor 224. The TX processing circuitry 214 encodes, multiplexes, and/or digitizes the outgoing baseband data to generate processed baseband or IF signals. The RF transceivers 209a-209n receive the outgoing processed baseband or IF signals from the TX processing circuitry 214 and up-converts the baseband or IF signals to RF signals that are transmitted via the antennas 204a-204n.

The controller/processor 224 can include one or more processors or other processing devices that control the overall operation of the AP 101. For example, the controller/ processor 224 could control the reception of uplink channel signals and the transmission of downlink channel signals by the RF transceivers 209a-209n, the RX processing circuitry 219, and the TX processing circuitry 214 in accordance with well-known principles. The controller/processor 224 could support additional functions as well, such as more advanced wireless communication functions. For instance, the controller/processor 224 could support beam forming or directional routing operations in which outgoing signals from multiple antennas 204a-204n are weighted differently to effectively steer the outgoing signals in a desired direction. The controller/processor 224 could also support OFDMA operations in which outgoing signals are assigned to different subsets of subcarriers for different recipients (e.g., different STAs 111-114). Any of a wide variety of other functions could be supported in the AP 101 by the controller/processor 224 including respiration rate detection using Wi-Fi. In some embodiments, the controller/processor 224 includes at least one microprocessor or microcontroller. The controller/processor 224 is also capable of executing programs and other processes resident in the memory 229, such as an OS. The controller/processor 224 can move data into or out of the memory 229 as required by an executing process.

The controller/processor 224 is also coupled to the backhaul or network interface 234. The backhaul or network interface 234 allows the AP 101 to communicate with other devices or systems over a backhaul connection or over a network. The interface 234 could support communications over any suitable wired or wireless connection(s). For example, the interface 234 could allow the AP 101 to communicate over a wired or wireless local area network or over a wired or wireless connection to a larger network (such as the Internet). The interface 234 includes any suitable structure supporting communications over a wired or wireless connection, such as an Ethernet or RF transceiver. The memory 229 is coupled to the controller/processor 224. Part of the memory 229 could include a RAM, and another part of the memory 229 could include a Flash memory or other ROM.

As described in more detail below, the AP 101 may include circuitry and/or programming for respiration rate detection using Wi-Fi. Although FIGURE 2A illustrates one example of AP 101, various changes may be made to FIGURE 2A. For example, the AP 101 could include any number of each component shown in FIGURE 2A. As a particular example, an access point could include a number of interfaces 234, and the controller/processor 224 could support routing functions to route data between different network addresses. As another particular example, while shown as including a single instance of TX processing circuitry 214 and a single instance of RX processing circuitry 219, the AP 101 could include multiple instances of each (such as one per RF transceiver). Alternatively, only one antenna and RF transceiver path may be included, such as in legacy APs. Also, various components in FIG. 2A could be combined, further subdivided, or omitted and additional components could be added according to particular needs.

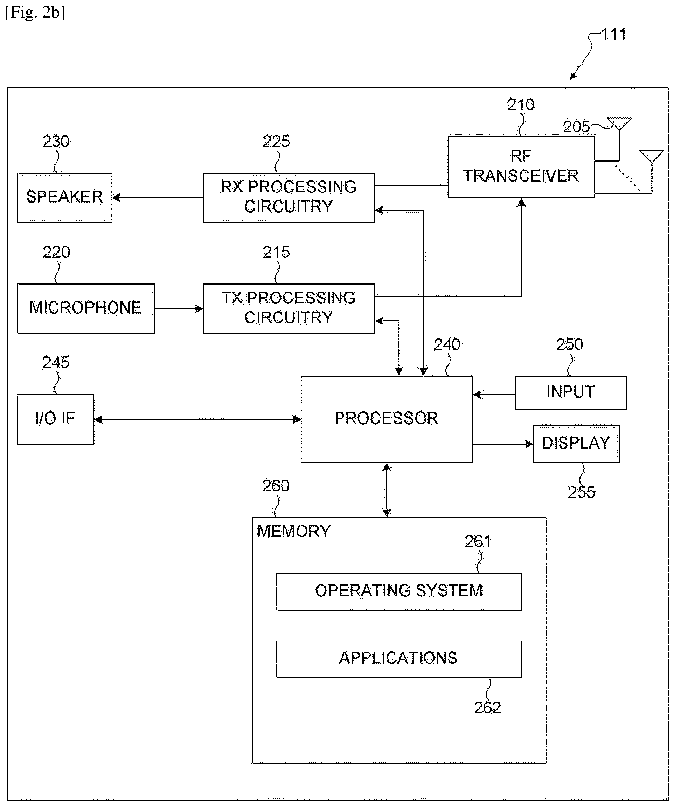

FIGURE 2B illustrates an example STA 111 according to embodiments of the present disclosure. The embodiment of the STA 111 illustrated in FIGURE 2B is for illustration only, and the STAs 112-114 of FIGURE 1 could have the same or similar configuration. However, STAs come in a wide variety of configurations, and FIGURE 2B does not limit the scope of this disclosure to any particular implementation of a STA.

The STA 111 includes antenna(s) 205, a radio frequency (RF) transceiver 210, TX processing circuitry 215, a microphone 220, and receive (RX) processing circuitry 225. The STA 111 also includes a speaker 230, a controller/processor 240, an input/output (I/O) interface (IF) 245, a touchscreen 250, a display 255, and a memory 260. The memory 260 includes an operating system (OS) 261 and one or more applications 262.

The RF transceiver 210 receives, from the antenna(s) 205, an incoming RF signal transmitted by an AP of the network 100. The RF transceiver 210 down-converts the incoming RF signal to generate an intermediate frequency (IF) or baseband signal. The IF or baseband signal is sent to the RX processing circuitry 225, which generates a processed baseband signal by filtering, decoding, and/or digitizing the baseband or IF signal. The RX processing circuitry 225 transmits the processed baseband signal to the speaker 230 (such as for voice data) or to the controller/processor 240 for further processing (such as for web browsing data).

The TX processing circuitry 215 receives analog or digital voice data from the microphone 220 or other outgoing baseband data (such as web data, e-mail, or interactive video game data) from the controller/processor 240. The TX processing circuitry 215 encodes, multiplexes, and/or digitizes the outgoing baseband data to generate a processed baseband or IF signal. The RF transceiver 210 receives the outgoing processed baseband or IF signal from the TX processing circuitry 215 and up-converts the baseband or IF signal to an RF signal that is transmitted via the antenna(s) 205.

The controller/processor 240 can include one or more processors and execute the basic OS program 261 stored in the memory 260 in order to control the overall operation of the STA 111. In one such operation, the main controller/processor 240 controls the reception of downlink channel signals and the transmission of uplink channel signals by the RF transceiver 210, the RX processing circuitry 225, and the TX processing circuitry 215 in accordance with well-known principles. The main controller/processor 240 can also include processing circuitry configured for respiration rate detection using Wi-Fi. In some embodiments, the controller/processor 240 includes at least one microprocessor or microcontroller.

The controller/processor 240 can move data into or out of the memory 260 as required by an executing process. In some embodiments, the controller/processor 240 is configured to execute a plurality of applications 262, such as applications for respiration rate detection using Wi-Fi. The controller/processor 240 can operate the plurality of applications 262 based on the OS program 261 or in response to a signal received from an AP. The main controller/processor 240 is also coupled to the I/O interface 245, which provides STA 111 with the ability to connect to other devices such as laptop computers and handheld computers. The I/O interface 245 is the communication path between these accessories and the main controller 240.

The controller/processor 240 is also coupled to the touchscreen 250 and the display 255. The operator of the STA 111 can use the touchscreen 250 to enter data into the STA 111. The display 255 may be a liquid crystal display, light emitting diode display, or other display capable of rendering text and/or at least limited graphics, such as from web sites. The memory 260 is coupled to the controller/processor 240. Part of the memory 260 could include a random access memory (RAM), and another part of the memory 260 could include a Flash memory or other read-only memory (ROM).

Although FIGURE 2B illustrates one example of STA 111, various changes may be made to FIGURE 2B. For example, various components in FIGURE 2B could be combined, further subdivided, or omitted and additional components could be added according to particular needs. In particular examples, the STA 111 may include any number of antenna(s) 205 for MIMO communication with an AP 101. In another example, the STA 111 may not include voice communication or the controller/ processor 240 could be divided into multiple processors, such as one or more central processing units (CPUs) and one or more graphics processing units (GPUs). Also, while FIGURE 2B illustrates the STA 111 configured as a mobile telephone or smartphone, STAs could be configured to operate as other types of mobile or stationary devices.

As discussed above, respiration rate detection plays an important role in various applications of health care, such as sleep apnea detection and early detection of chronic respiratory diseases. Conventional respiration rate detection techniques require patients to wear devices such as nasal tubes, capnometers, or chest pressure sensors, which are expensive and uncomfortable to wear. To enable contact-free respiration rate detection, recently respiration monitoring with Doppler radar, UWB radar and FMCW radar have been proposed. Although these radar systems allow relatively precise measurements, they are very expensive due to the cost of dedicated hardware.

Wi-Fi sensing based respiration rate detection, which detects respiration rate using the channel state information (CSI) from ubiquitous Wi-Fi infrastructure and commercial off-the-shelf (COTS) Wi-Fi devices, has attracted interest in both industry and academia in recent years. To be specific, when an AP is communicating with a STA, the CSI represents how Wi-Fi signals propagate from the AP to the STA at certain carrier frequencies along multiple paths. Since CSI reflects how Wi-Fi signals travel through surrounding objects and humans in time, frequency, and spatial domains, detecting CSI changes can capture the periodic movements of a human chest due to breathing.

Since any room is often occupied by multiple people, a key function for the respiration rate detection system is to detect respiration rates of multiple people in the same room simultaneously, which has rarely been addressed. Some conventional techniques rely on multiple Wi-Fi transmitter (TX) and receiver (RX) pairs for this challenging task. However, it is impractical to deploy multiple dedicated Wi-Fi devices in a room only for detecting respiration rates. Therefore, how to detect the respiration rates of multiple people with only one Wi-Fi TX-RX pair is an un-addressed but important challenge.

To address these and other issues, this disclosure provides systems and methods for respiration rate detection using Wi-Fi. As described in more detail below, the disclosed embodiments use Wi-Fi CSI to estimate respiration rates of multiple people with a single COTS Wi-Fi transmitter and receiver pair. In other words, multiple pairs of Wi-Fi transmitters and receivers are not needed in the disclosed embodiments. In the disclosed embodiments, signal processing is used to clean noisy Wi-Fi CSI on the single transmitter-receiver pair. Then, a number of distinct respiration rates are detected based on the clean CSI data. In some embodiments, the respiration rates of multiple people can be detected at a relatively far distance.

Note that while some of the embodiments discussed below are described in the context of consumer Wi-Fi enabled devices such as smart phones, tablets, and televisions, these are merely examples. It will be understood that the principles of this disclosure may be implemented in any number of other suitable contexts or systems.

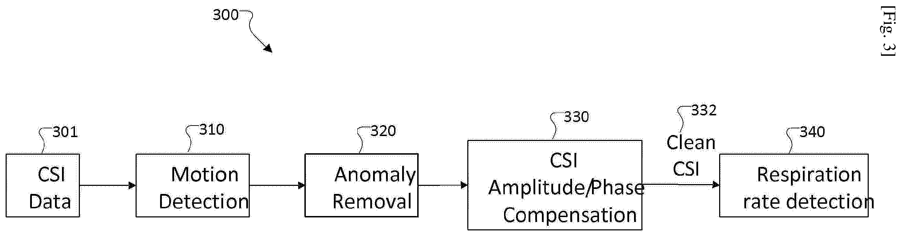

FIGURE 3 illustrates an example process 300 for respiration rate detection using Wi-Fi according to embodiments of the present disclosure. The embodiment of the process 300 shown in FIGURE 3 is for illustration only. Other embodiments of the process 300 could be used without departing from the scope of this disclosure. For ease of explanation, the process 300 will be described as being implemented in the STA 111 of FIGURE 1. However, the process 300 could be implemented in any other suitable device.

As shown in FIGURE 3, the process 300 starts with the STA 111 obtaining CSI data 301 as input to the process 300. The CSI data is from a single Wi-Fi transmitter-receiver pair and is obtained regularly or continuously over a time period. The transmitter of the transmitter-receiver pair may be, e.g., a home Wi-Fi router. The transmitter may represent (or be represented by) the AP 101 of FIGURE 1. The receiver of the transmitter-receiver pair is located in a Wi-Fi coverage area of the transmitter, and is wirelessly connected to the transmitter, such that Wi-Fi signals are transmitted between the transmitter and the receiver. In some embodiments, the receiver may be, e.g., a smart phone or tablet. The receiver may represent (or be represented by) the STA 111.

The STA 111 performs a motion detection operation 310 on the CSI data 301. The motion detection operation 310 is performed to remove any large variations in the CSI data 301 caused by environment changes, such as movement of people or objects in the Wi-Fi coverage area of the transmitter. This is because the process 300 is performed optimally in relatively stationary scenarios (i.e., minimal movement of people or objects in the Wi-Fi coverage area of the transmitter). During the motion detection operation 310, if the STA 111 detects large motion in the CSI data 301, then the STA 111 discards the signal. Otherwise, if the STA 111 does not detect large motion in the CSI data 301, then the STA 111 provides the signal as input to the next operation. Any suitable algorithm or technique can be used for the motion detection operation 310.

After the motion detection operation 310 is performed, the STA 111 performs an anomaly removal operation 320 to remove anomalies (e.g., wrong or abnormal CSI types) from the CSI data 301. It is observed that different time samples of the CSI data 301 can exhibit very different shapes of CSI amplitude over different subcarriers. As an example, such abnormal CSI can be due to the transmitter's antenna switching.

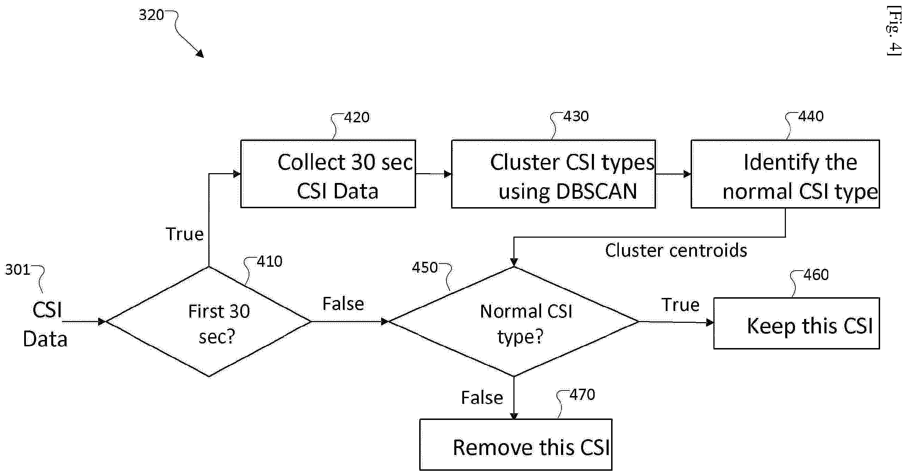

FIGURE 4 illustrates an example of the anomaly removal operation 320 according to embodiments of the present disclosure. As shown in FIGURE 4, the anomaly removal operation 320 is a low complexity algorithm that includes use of the DBSCAN clustering algorithm.

At operation 410, it is determined if the current sample of CSI data 301 is within the first thirty seconds of CSI data obtained after the start of the process 300. The first thirty seconds of CSI data 301 is used to establish a "normal" CSI type for the transmitter-receiver pair. If the current sample of CSI data 301 is within the first thirty seconds of CSI data, then at operation 420, the STA 111 collects the CSI data 301 until thirty seconds of CSI data 301 is collected. At operation 430, the STA 111 uses the DBSCAN algorithm on the CSI data 301 to create clusters for different CSI types (e.g., using cosine similarity as the distance metric). The DBSCAN algorithm decides how many clusters or categories are represented in the CSI data 301, and determines the centroid of each cluster.

At operation 440, the STA 111 uses the clusters to identify the normal CSI type for the transmitter-receiver pair. In some embodiments, the cluster with the greatest number of samples is identified as the "normal" CSI type, while other clusters are identified as "abnormal" or "wrong" CSI types. In other embodiments, the cluster i is identified as the normal cluster if its centroid CSI $CSI_{core,i}$ has the smoothest variation across sub-carriers, i.e., lowest delay spread.

For incoming samples of CSI data 301 after the first thirty seconds, the STA 111 performs operation 450 to compare the newly-obtained CSI data sample 301 with the existing DBSCAN-based clusters and determine if the newly-obtained CSI data sample 301 is of the normal CSI type (e.g., assigned to the normal cluster). For example, each newly obtained CSI data sample 301, CSInew, is assigned to a cluster, if its distance from the centroid of cluster i is smaller than its distance from other cluster centroids. In one embodiment, this distance function f(CSInew,CSIcore,i) used for clustering can be the cosine distance between the CSI centroid CSIcore,i of each cluster i and the new CSI data sample CSInew. Each new CSI data sample 301 assigned to the normal cluster is kept and stored in a buffer (operation 460). If a new CSI data sample 301 is not assigned to the normal cluster, the CSI data sample 301 is dropped as an anomaly (operation 470). In one variant, a CSI data sample 301 assigned to the normal cluster can be discarded if its distance from the normal cluster centroid is higher than a threshold.

Returning to FIGURE 3, after the anomaly removal operation 320 is performed, the STA 111 extracts the amplitude and phase from the normal CSI data 301 and then performs a CSI compensation operation 330 to accomplish CSI amplitude and/or phase compensation. As described below, the CSI compensation operation 330 removes any sampling time offset(s), which can cause large phase error.

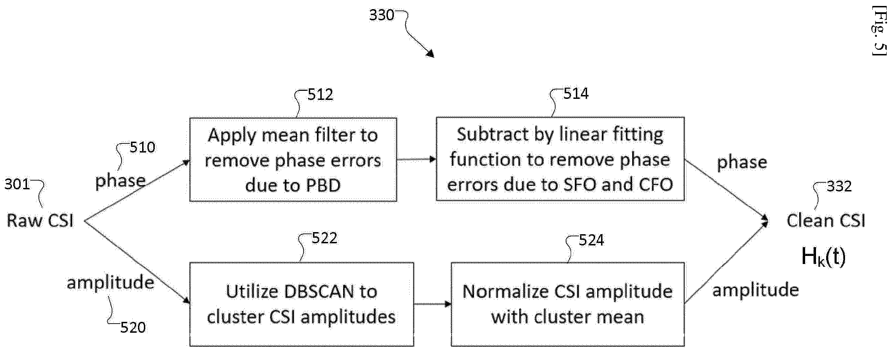

FIGURE 5 illustrates an example of the CSI compensation operation 330 according to embodiments of the present disclosure. As shown in FIGURE 5, the CSI compensation operation 330 includes two low complexity algorithms-phase compensation 510 and amplitude compensation-that can be performed on the CSI data 301 in real time to generate clean CSI data 332.

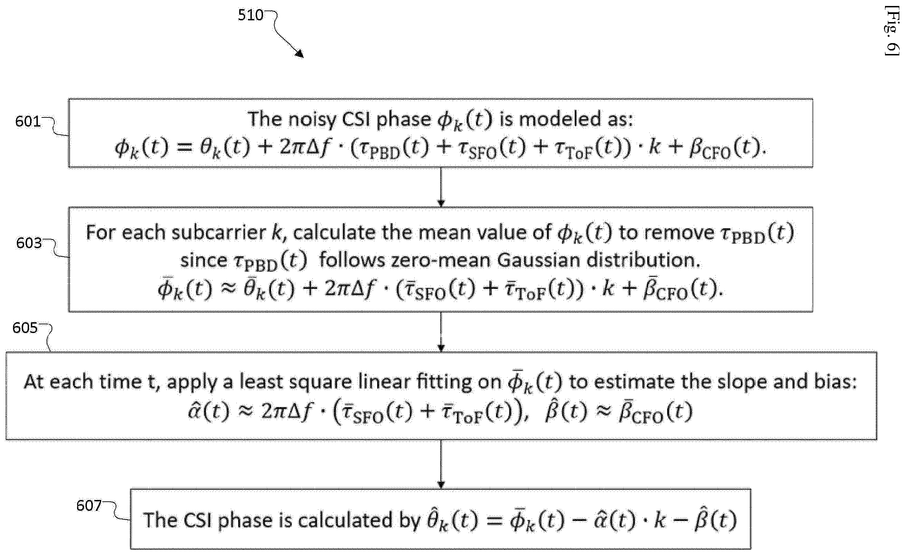

The phase compensation operation 510 is a low complexity algorithm that includes a mean filtering operation 512 and a linear fitting operation 514. FIGURE 6 illustrates further details of the phase compensation operation 510 according to embodiments of the present disclosure. As shown in FIGURE 6, the phase compensation operation 510 includes operations 601-607, where operations 601 and 603 generally correspond to the mean filtering operation 512, and operations 605 and 607 generally correspond to the linear fitting operation 514.

At operation 601, the mathematical model of the noisy CSI phase on subcarrier k of time t can be represented as:

$$(1) \quad \Phi_k(t) = \Theta_k(t) = 2\Pi \Delta f^* (T_{PBD}(t) + T_{SFO}(t) + T_{T0F}(t))^* k + B_{CFO}(t)$$

where $\Theta_k(t)$ is the clean CSI phase, $T_{PBD}(t)$ is the delay cause by packet boundary detection (PBD), $T_{SFO}(t)$ is the delay cause by sampling frequency offset (SFO), and $B_{CFO}(t)$ is the frequency offset caused by central frequency offset (CFO).

At operation 603, for each subcarrier k, the STA 111 applies a mean filter to $\Theta_k(t)$ to remove $T_{PBD}(t)$. The STA 111 applies the mean filter to remove phase errors due to $T_{PBD}(t)$ since $T_{PBD}(t)$ follows zero-mean Gaussian distribution.

At operation 605, for each time t, the STA applies a least square linear tting to $\Theta_k(t)$ to estimate $T_{SFO}(t)$ and $T_{T0F}(t)$.

At operation 607, the STA 111 removes phase errors due to $T_{SFO}(t)$ and $B_{CFO}(t)$ by subtracting a fitted linear function. The clean CSI phase can be obtained by removing the linear fitting function from operation 605. It is important to note that most convention techniques remove CSI phase errors using the CSI ratio, which requires two TX-RX links. In contrast, the phase compensation operation 510 uses only a single TX-RX link. That is, only a single antenna pair is needed.

Returning to FIGURE 5, the amplitude compensation operation 520 is performed to remove fluctuation in the CSI amplitude, thereby normalizing the CSI amplitude. The CSI amplitude fluctuates over time due to the imperfect automatic gain controller (AGC) component on the STA 111's hardware. The amplitude compensation operation 520 is a low complexity algorithm that includes a clustering operation 522 and a normalization operation 524.

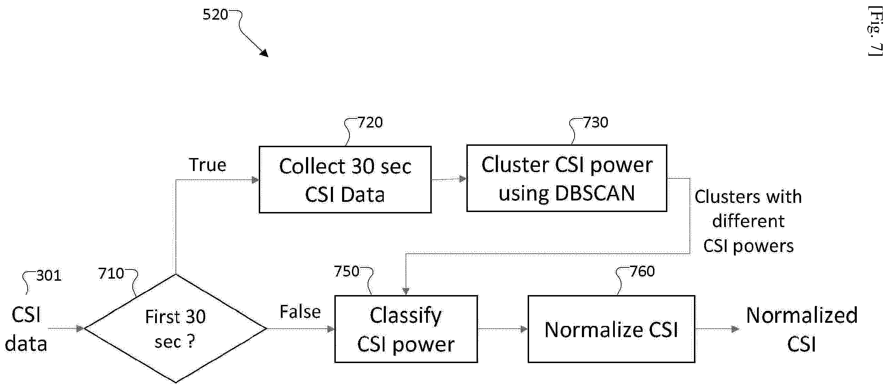

FIGURE 7 illustrates further details of the amplitude compensation operation 520 according to embodiments of the present disclosure. As shown in FIGURE 7, the amplitude compensation operation 520 is a low complexity algorithm that includes use of the DBSCAN clustering algorithm. The amplitude compensation operation 520 includes operations 710-760, where operations 720 and 730 generally correspond to the mean clustering operation 522, and operations 750 and 760 generally correspond to the normalization operation 524.

At operation 710, it is determined if the current sample of CSI data 301 is within the first thirty seconds of CSI data. The first thirty seconds of CSI data 301 is used to establish a baseline power for the transmitter-receiver pair. If the current sample of CSI data 301 is within the first thirty seconds of CSI data, then at operation 720, the STA 111 collects the CSI data 301 until thirty seconds of CSI data 301 is collected. At operation 730, the STA 111 uses the DBSCAN algorithm on the CSI data 301 to create clusters for different CSI types (e.g., using cosine similarity as the distance metric). The DBSCAN algorithm decides how many clusters or categories are represented in the CSI data 301, and determines the centroid of each cluster, i.e., the total energy of the CSI data 301 across all subcarriers. Then each CSI data 301 belonging to the first thirty seconds is normalized by dividing by the mean CSI power of its associated cluster.

For incoming samples of CSI data 301 after the first thirty seconds, the STA 111 performs operation 750 to classify the CSI power of the newly-obtained CSI data sample 301. For example, each newly obtained CSI data sample 301, CSInew, is assigned to a cluster i, if its distance from the centroid of cluster i is smaller than its distance from other cluster centroids. In one embodiment, this distance function used for clustering can be the absolute difference between the CSI power of CSInew, and the mean CSI power of each cluster i. Then, in operation 760, the STA 111 normalizes this CSI data sample 301 by dividing by the mean CSI power of its assigned cluster. The clean CSI 332 (after amplitude and phase compensation) on subcarrier k and time index t can be represented as Hk(t).

Returning to FIGURE 3, after the CSI compensation operation 330 is performed and the clean CSI 332 is obtained, the STA 111 performs a multi-person respiration rate detection operation 340 using the clean CSI 332 as an input. The STA 111 performs the multi-person respiration rate detection operation 340 to distinguish distinct respiration rates of multiple people in the clean CSI 332. In the operation 340, the sub-carriers that contain respiration signals are selected based on large respiration energy ratio (RER) values. Next, the FFT peaks corresponding to different people's respiration rates are identified. Then, for each identified respiration rate, an algorithm is performed to detect the number of people that have the same respiration rate. Lastly, each respiration rate is classified to different people based on the peoples' respiration rate histories. As described below, the multi-person respiration rate detection operation 340 is low-complexity and can be executed in real time.

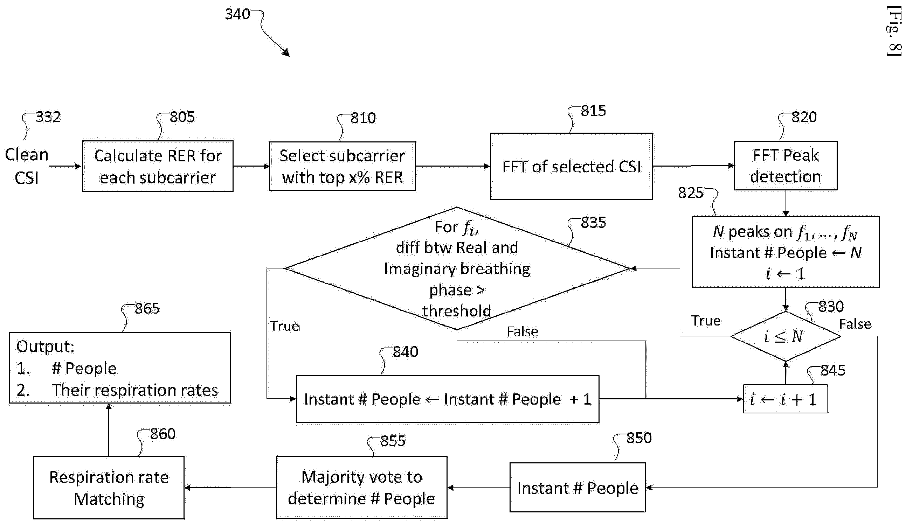

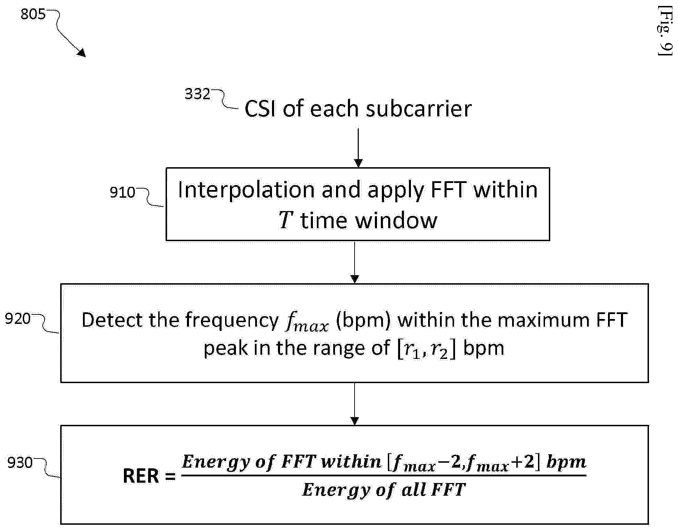

FIGURE 8 illustrates an example of the multi-person respiration rate detection operation 340 according to embodiments of the present disclosure. As shown in FIGURE 8, the STA 111 obtains the clean CSI data 332 as input and performs operation 805 to calculate RER values for each CSI subcarrier. FIGURE 9 illustrates further details of the operation 805. As shown in FIGURE 9, at operation 910, the STA 111 interpolates and applies FFT to each CSI subcarrier. At operation 920, the STA 111 detects the FFT peak fmax that has the maximum amplitude within [r1,r2], where [r1,r2] represents a normal range for human respiration rate, in breaths per minute (bpm). Then, at operation 930, the STA 111 calculates the RER for each subcarrier, such as by using the following equation:

$$(2) \quad RER = \frac{Energy\,of\,FFT\,within\,[fmax-2,fmax+2]\,bpm}{Energy\,of\,all\,FFT}$$

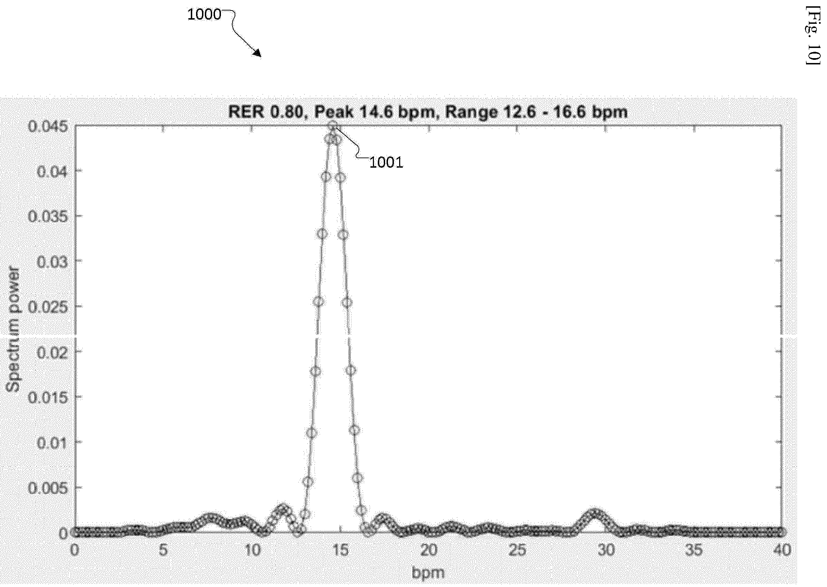

Once the RER values are calculated, the STA 111 performs a subcarrier selection operation 810. In some embodiments, the STA 111 selects subcarriers that contain strong reflected signals from human respiration movement based the calculated RER values. For example, the STA 111 can sort the subcarriers by their corresponding RER values, and select the x% of subcarriers with the largest RER values, since these sub-carriers may carry strong reflected signals from one or more human's breathing movements. Here, the value of x may be empirically determined. As an example, FIGURE 10 illustrates a chart 1000 showing the FFT of one subcarrier. As shown in FIGURE 10, the chart 1000 includes a FFT peak 1001 around 14.6 bpm. Since the subcarrier shown in FIGURE 10 includes such a strong FFT peak (and thus a large RER value), the subcarrier would likely be included among the selected subcarriers.

Once the subcarriers are selected, the STA 111 obtains the FFT of the selected CSI at operation 815, and then performs a FFT peak detection operation 820 to determine the number of distinct respiration rates, f1,f2...fN, which are associated with the FFT peaks. The clean CSI 332 on selected subcarrier k of time t can be approximated as:

$$(3) \quad Hk(t) = B_k + \sum_{n=1}^{s} jc\, A_{n,k} \sin(2\Pi f_{br,n}(t) + \Phi n)$$

$f_{br,n}(t), \Phi_n$ are the breathing frequency and phase of person n, and s is the total number of people in the environment around the transmitter-receiver pair.

From Equation (3), it can be seen that Hk(t) has a component that is periodic with fbr,n for each n. In one embodiment, the human respiration rate is expected to lie within a frequency range of[fbr,min , tbr,max] . Thus, the peaks of the Doppler power spectrum of Hk(t) for selected subcarriers k, that lie within the human respiration frequency range, are used to detect the distinct respiration rates. However, multiple people may have the same respiration rate. In some embodiments, the selection threshold is based on the frequency resolution, which is the inverse of the input time window.

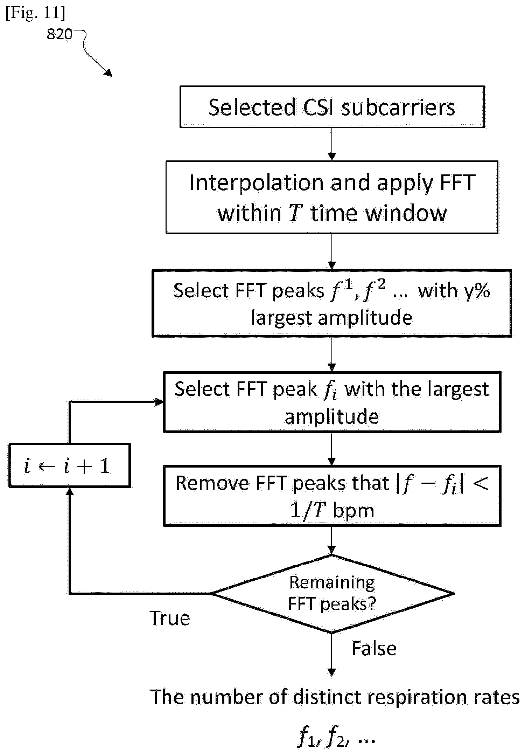

FIGURE 11 illustrates further details of the FFT peak detection operation 820. As shown in FIGURE 11, large FFT peaks are selected and clustered based on the frequency resolution, i.e., the FFT peaks with frequency difference less than the frequency resolution are classified as the same cluster. For each cluster, the FFT peak with the maximum amplitude is the detected respiration rate of that cluster. The number of clusters represents the number of distinct respiration rates. It is important to note that multiple people may have the same respiration rate, so the number of distinct respiration rates may not be equal to the number of people in the vicinity.

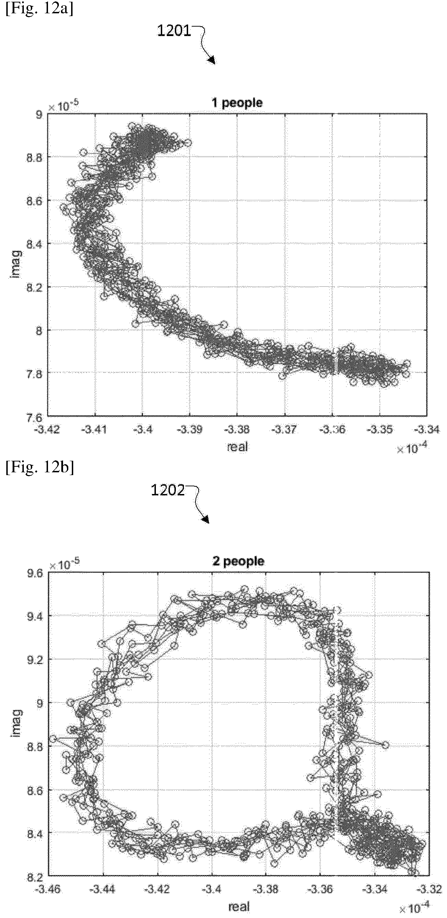

Once the STA 111 determines the distinct respiration rates f1,f2...fN, the STA 111 performs an iterative loop (operations 825-845) to detect the number of people represented by the distinct respiration rates. This can account for the fact that two or more people in the vicinity of the transmitter-receiver pair may have the same respiration rate. To be specific, when two users share the same respiration rate, i.e., for s=2 and f br1=f br2, note from Equation (3) that the trajectory followed by Hk(t) in the complex plane with varying t is approximately a sum of two sinusoids, i.e., an ellipse. On the other hand, for s=1, the trajectory in the complex plane is approximately an arc. This difference in CSI trajectory for s=1 and s=2 is depicted in FIGURES 12A and 12B. FIGURE 12A shows a chart 1201 of the arc-shaped CSI trajectory for s=1, while FIGURE 12B shows a chart 1202 of the generally ellipse-shaped CSI trajectory for s=2. This difference in shape of the CSI trajectories can be exploited to separate the one person case from the multi-person cases.

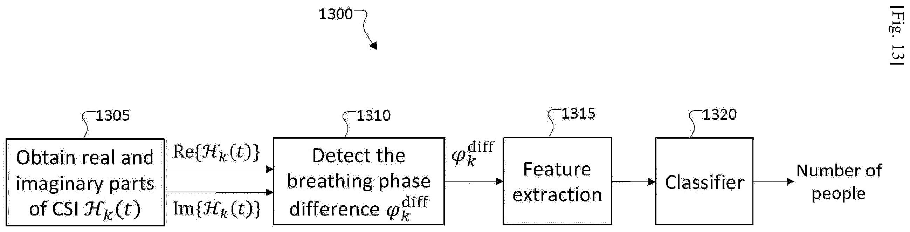

FIGURE 13 illustrates a method 1300 of counting people with the same respiration rate according to embodiments of the present disclosure. As shown in FIGURE 13, at operation 1305, the clean CSI 332 is filtered, and the filtered CSI data is projected along real and imaginary axes, i.e., Re{Hk(t)} and Im{Hk(t)} in order to obtain the real and imaginary parts of Hk(t). At operation 1310, the breathing phase difference, between the real and imaginary signals is detected. For example, the breathing phase difference can be detected using the following equation:

$$(4) \quad \Phi_k^{diff} = \arg\cos\left( \frac{[Re(Hk(0)),\ldots RE(Hk(t))] \cdot [Im(Hk(0)),\ldots Im(Hk(t))]}{||[Re(Hk(0)),\ldots RE(Hk(t))]|| \cdot ||[Im(Hk(0)),\ldots Im(Hk(t))]||} \right).$$

If the CSI trajectory is an arc, $\Phi_k^{diff}$ is equal to or close to 0 or $\pi$. On the other hand, if the CSI trajectory is an ellipse, $\Phi_k^{diff}$ is equal to or close to $\pi/2$ or $3\pi/2$.

According to this characteristic, features are extracted at operation 1315. In some embodiments, $\left|\sin(\Phi_k^{diff})\right|$ is utilized as the feature to detect the number of people since $\left|\sin(\Phi_k^{diff})\right| = 0$ under single-person scenario and $\left|\sin(\Phi_k^{diff})\right| = 1$ under multi-person scenario.

Then, at operation 1320, a low complexity classifier is trained to detect the number of people of each distinct respiration rate using the median of $\left|\sin(\Phi_k^{diff})\right|$ over selected subcarriers as the input feature of the classifier. In some embodiments, this low complexity classifier is a linear classifier with input of $\left|\sin(\Phi_k^{diff})\right|, \left|\cos(\Phi_k^{diff})\right|$ over each selected subcarrier and antenna. In other embodiments, statistics functions of $\left|\sin(\Phi_k^{diff})\right|, \left|\cos(\Phi_k^{diff})\right|$ over subcarriers and antennas are used. In some embodiments, a median function is used on this metric over all selected subcarriers and antennas. In other embodiments, a machine learning (ML) network (e.g., a neural network) is used to distinguish the one person and two people cases. The input to the ML network is the cosine and sine function of the breathing phase difference, and the output of the ML network is the one person and two people detection results.

Returning to FIGURE 8, the output of the people counting loop is an instant number 850 of people (e.g., one person having the particular respiration rate, or two (or more) people having the particular respiration rate). The instant number 850 reflects clean CSI data 332 at one particular time period. It is possible that the number of people detected can change over time. For example, the instant number 850 may be determined at one-second intervals, and may occasionally be one value (e.g., one person) and may occasionally be another value (e.g., two people). To account for any possible differences, the STA 111 can take all of the determined instant numbers 850 over a time period and use a majority vote operation 855 to make a final determination of the number of people.

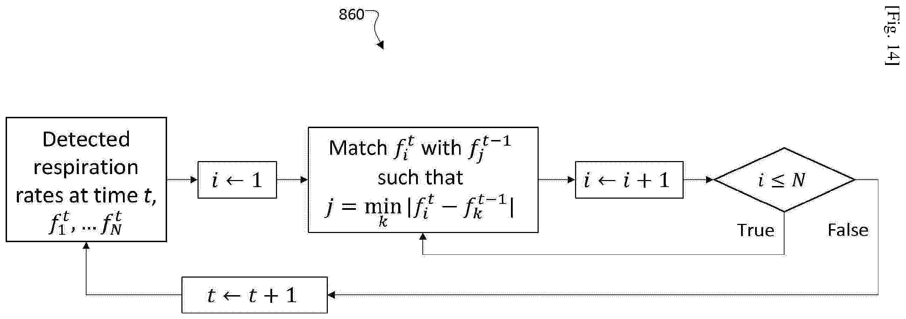

After obtaining the respiration rates and the number of people, the STA 111 performs a respiration rate matching operation 860 to assign each respiration rate to the different people. The respiration rate matching operation 860 is performed based on people's respiration rate histories. FIGURE 14 illustrates further details of the respiration rate matching operation 860. As shown in FIGURE 14, the respiration rate matching operation 860 is a low complexity rate matching method. Since a person's respiration rate can be assumed to be quasi-static, at time t, each respiration rate is classified as one person's respiration rate if the absolute difference of each respiration rate and that person's respiration rate at time t-1 is the smallest among all people.

After the STA 111 performs the respiration rate matching operation 860 to assign each respiration rate to the different people, the STA 111 generates an output 865 for the multi-person respiration rate detection operation 340. In some embodiments, the output 865 includes the number of people detected in the vicinity and their particular respiration rates.

Although FIGURES 3 through 14 illustrate an example of a process 300 for respiration rate detection using Wi-Fi and related details, various changes may be made to FIGURES 3 through 14. For example, while various disclosed operations are described as using DBSCAN, other algorithms could be used. As another example, while FIGURES 4 and 7 describe thirty second periods of time for establishing a baseline, other periods of time could be used. Also, various components in FIGURES 3 through 14 could be combined, further subdivided, or omitted and additional components could be added according to particular needs. In addition, various operations in FIGURES 3 through 14 could overlap, occur in parallel, occur in a different order, or occur any number of times.

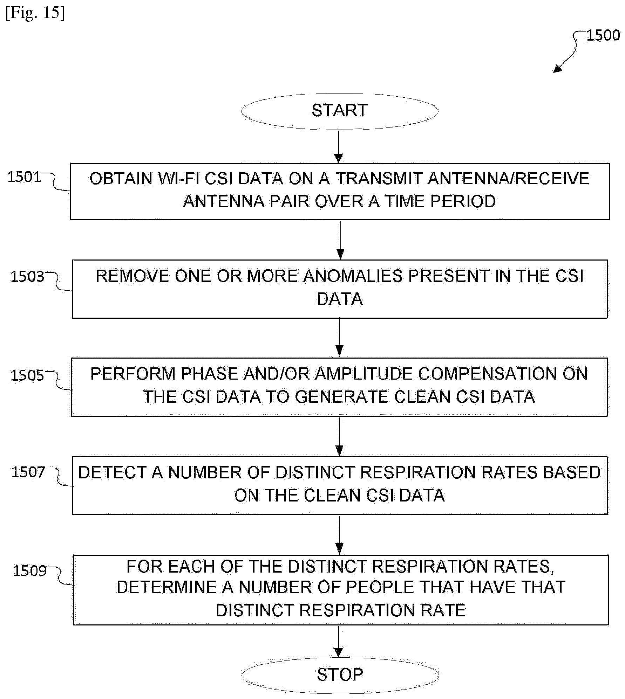

FIGURE 15 illustrates a flow chart of a method 1500 for respiration rate detection using Wi-Fi according to embodiments of the present disclosure, as may be performed by a STA (e.g., the STA 111). The embodiment of the method 1500 shown in FIGURE 15 is for illustration only. One or more of the components illustrated in FIGURE 15 can be implemented in specialized circuitry configured to perform the noted functions, or one or more of the components can be implemented by one or more processors executing instructions to perform the noted functions.

As illustrated in FIGURE 15, the method 1500 begins at step 1501. At step 1501, a STA obtains Wi-Fi CSI data on a transmit antenna/receive antenna pair over a time period. This could include, for example, the STA 111 obtaining the CSI data 301 on a transmit antenna/receive antenna pair over a time period, such as described with respect to FIGURE 3.

At step 1503, the STA removes one or more anomalies present in the CSI data. This could include, for example, the STA 111 removing one or more anomalies present in the CSI data 301, such as described with respect to FIGURE 4.

At step 1505, the STA performs at least one of phase compensation and amplitude compensation on the CSI data to generate clean CSI data. This could include, for example, the STA 111 performing the phase compensation operation 510, the amplitude compensation operation 520, or both, on the CSI data 301 to generate clean CSI data 332, such as described with respect to FIGURES 5 through 7.

At step 1507, the STA detects a number of distinct respiration rates based on the clean CSI data. This could include, for example, the STA 111 detecting a number of distinct respiration rates f1,f2...fN based on the clean CSI data 332, such as described with respect to FIGURE 11.

At step 1509, the STA determines, for each of the distinct respiration rates, a number of people that have that distinct respiration rate. This could include, for example, the STA 111 determining a number of people that have each of the distinct respiration rates f1,f2...fN, such as described with respect to FIGURE 13.

Although FIGURE 15 illustrates one example of a method 1500 for respiration rate detection using Wi-Fi, various changes may be made to FIGURE 15. For example, while shown as a series of steps, various steps in FIGURE 15 could overlap, occur in parallel, occur in a different order, or occur any number of times.

Although the present disclosure has been described with exemplary embodiments, various changes and modifications may be suggested to one skilled in the art. It is intended that the present disclosure encompass such changes and modifications as fall within the scope of the appended claims. None of the description in this application should be read as implying that any particular element, step, or function is an essential element that must be included in the claims scope. The scope of patented subject matter is defined by the claims.

**Claims**

1. A computer-implemented method to detect respiration rates of multiple people in the same room simultaneously, comprising:

   obtaining Wi-Fi channel state information, CSI, data on a transmit antenna/receive antenna pair over a time period (1501);
   removing one or more anomalies present in the CSI data (1503);
   performing at least one of phase compensation and amplitude compensation on the CSI data to generate clean CSI data (1505);
   detecting a number of distinct respiration rates based on the clean CSI data (1507); and
   for each of the distinct respiration rates, determining the number of people that have that distinct respiration rate (1509),
   **characterised in that** removing the one or more anomalies present in the CSI data comprises:

   collecting CSI data for a predetermined time period;
   clustering CSI types within the CSI data collected during the predetermined time period using a clustering algorithm;
   identifying a normal CSI type based on the clustered CSI types; and
   removing a portion of CSI data received after the predetermined time period that does not correspond to the normal CSI type.

2. The method of Claim 1, wherein performing the phase compensation on the CSI data (1505) comprises:

   applying a mean filter to remove one or more phase errors due to packet boundary detection error; and
   subtracting a fitted linear function to remove one or more other phase errors due to (i) a delay caused by a sampling frequency offset and (ii) a frequency offset caused by central frequency offset.

3. The method of Claim 1, wherein performing the amplitude compensation on the CSI data (1505) comprises:

   clustering amplitudes of the CSI data using a clustering algorithm; and
   normalizing the CSI data by dividing the CSI data by a mean CSI amplitude of a corresponding CSI data cluster.

4. The method of Claim 1, wherein detecting the number of distinct respiration rates based on the clean CSI data (1507) comprises:

   selecting subcarriers containing respiration signals based on respiration energy ratio, RER, values obtained from the clean CSI data;
   identifying fast Fourier transform, FFT, peaks corresponding to respiration rates of different people; and
   setting the number of identified FFT peaks as the number of distinct respiration rates.

5. The method of Claim 4, wherein determining the number of people that have that distinct respiration rate (1509) comprises:
   for each of the identified FFT peaks:

   filtering the clean CSI data to obtain filtered CSI data corresponding to that identified FFT peak;
   determining real and imaginary parts of the filtered CSI data in a complex plane;
   detecting a breathing phase difference between the real and imaginary parts; and
   determining the number of people that have that distinct respiration rate based on the breathing phase difference.

6. The method of Claim 5, wherein determining the number of people that have that distinct respiration rate (1509) comprises:
   determining the number of people based on a continuous series of instant detection results of numbers of people.

7. A device comprising:

   a transceiver configured to receive Wi-Fi channel state information, CSI, data on a transmit antenna/receive antenna pair over a time period (1501); and

a processor operably connected to the transceiver, the processor configured to:

remove one or more anomalies present in the CSI data (1503);
perform at least one of phase compensation and amplitude compensation on the CSI data to generate clean CSI data (1505);
detect a number of distinct respiration rates based on the clean CSI data (1507); and
for each of the distinct respiration rates, determine a number of people that have that distinct respiration rate (1509),
**characterised in that** to remove the one or more anomalies present in the CSI data, the processor is configured to:

collecting CSI data for a predetermined time period;
cluster CSI types within the CSI data collected during the predetermined time period using a clustering algorithm;
identify a normal CSI type based on the clustered CSI types; and
remove a portion of the CSI data received after the predetermined time period that does not correspond to the normal CSI type.

8. The device of Claim 7, wherein to perform the phase compensation on the CSI data (1505), the processor is configured to:

apply a mean filter to remove one or more phase errors due to packet boundary detection error; and
subtract a fitted linear function to remove one or more other phase errors due to (i) a delay caused by a sampling frequency offset and (ii) a frequency offset caused by central frequency offset.

9. The device of Claim 7, wherein to perform the amplitude compensation on the CSI data (1505), the processor is configured to:

cluster amplitudes of the CSI data using a clustering algorithm; and
normalize the CSI data by dividing the CSI data by a mean CSI amplitude of a corresponding CSI data cluster.

10. The device of Claim 7, wherein to detect the number of distinct respiration rates based on the clean CSI data (1507), the processor is configured to:

select subcarriers containing respiration signals based on respiration energy ratio, RER, values obtained from the clean CSI data;
identify fast Fourier transform, FFT, peaks corresponding to respiration rates of different people; and
set the number of identified FFT peaks as the number of distinct respiration rates.

11. The device of Claim 10, wherein to determine the number of people that have that distinct respiration rate (1509), the processor is configured to:
for each of the identified FFT peaks:

filter the clean CSI data to obtain filtered CSI data corresponding to that identified FFT peak;
determine real and imaginary parts of the filtered CSI data in a complex plane;
detect a breathing phase difference between the real and imaginary parts; and
determine the number of people that have that distinct respiration rate based on the breathing phase difference.

12. The device of Claim 11, wherein the processor is configured to determine the number of people that have that distinct respiration rate (1509) based on a continuous series of instant detection results of numbers of people.

13. A non-transitory computer readable medium comprising program code that, when executed by a processor of a device, causes the device to:

obtain Wi-Fi channel state information, CSI, data on a transmit antenna/receive antenna pair over a time period (1501);
remove one or more anomalies present in the CSI data (1503);
perform at least one of phase compensation and amplitude compensation on the CSI data to generate clean CSI data (1505);
detect a number of distinct respiration rates based on the clean CSI data (1507); and
for each of the distinct respiration rates, determine a number of people that have that distinct respiration rate (1509),
**characterised in that** removing the one or more anomalies present in the CSI data comprises:

collecting CSI data for a predetermined time period;
clustering CSI types within the CSI data collected during the predetermined time period using a clustering algorithm;
identifying a normal CSI type based on the clustered CSI types; and
removing a portion of CSI data received after the predetermined time period that does not correspond to the normal CSI type.

**Patentansprüche**

1. Computerimplementiertes Verfahren zum gleichzeitigen Detektieren von Atmungsraten mehrerer Personen in demselben Raum, umfassend:

Erlangen von Kanalzustandsinformationsdaten, CSI-Daten, von Wi-Fi auf einem Sendeantenne/Empfangsantenne-Paar über einen Zeitraum (1501);
Entfernen einer oder mehrerer Anomalien, die in den CSI-Daten vorhanden sind (1503);
Durchführen von mindestens einem von Phasenkompensation und Amplitudenkompensation an den CSI-Daten, um saubere CSI-Daten (1505) zu generieren;
Detektieren einer Anzahl von unterschiedlichen Atmungsraten basierend auf den sauberen CSI-Daten (1507); und
für jede der unterschiedlichen Atmungsraten Bestimmen der Anzahl von Personen, die diese unterschiedliche Atmungsrate aufweisen (1509),
**dadurch gekennzeichnet, dass**
das Entfernen der einen oder der mehreren Anomalien, die in den CSI-Daten vorhanden sind, Folgendes umfasst:

Sammeln von CSI-Daten für einen vorbestimmten Zeitraum;
Clustern von CSI-Typen innerhalb der während des vorbestimmten Zeitraums gesammelten CSI-Daten unter Verwendung eines Clustering-Algorithmus;
Identifizieren eines normalen CSI-Typs basierend auf den geclusterten CSI-Typen; und
Entfernen eines Teils von CSI-Daten, der nach dem vorbestimmten Zeitraum empfangen wurde und nicht dem normalen CSI-Typ entspricht.

2. Verfahren nach Anspruch 1, wobei das Durchführen der Phasenkompensation an den CSI-Daten (1505) Folgendes umfasst:

Anwenden eines Mittelwertfilters, um einen oder mehrere Phasenfehler aufgrund eines Paketgrenzendetektionsfehlers zu entfernen; und
Subtrahieren einer angepassten linearen Funktion, um einen oder mehrere andere Phasenfehler aufgrund (i) einer Verzögerung, die durch einen Abtastfrequenzversatz verursacht wird, und (ii) eines Frequenzversatzes, der durch einen Mittenfrequenzversatz verursacht wird, zu entfernen.

3. Verfahren nach Anspruch 1, wobei das Durchführen der Amplitudenkompensation an den CSI-Daten (1505) Folgendes umfasst:

Clustern von Amplituden der CSI-Daten unter Verwendung eines Clustering-Algorithmus; und
Normalisieren der CSI-Daten durch Dividieren der CSI-Daten durch eine mittlere CSI-Amplitude eines entsprechenden CSI-Datenclusters.

4. Verfahren nach Anspruch 1, wobei das Detektieren der Anzahl von unterschiedlichen Atmungsraten basierend auf den sauberen CSI-Daten (1507) Folgendes umfasst:

Auswählen von Subträgern, die Atmungssignale enthalten, basierend auf Atmungsenergieverhältniswerten, RER-Werten, die aus den sauberen CSI-Daten erlangt werden;
Identifizieren von Peaks einer schnellen Fourier-Transformation, FFT-Peaks, die Atmungsraten verschiedener Personen entsprechen; und
Festlegen der Anzahl von identifizierten FFT-Peaks als die Anzahl von unterschiedlichen Atmungsraten.

5. Verfahren nach Anspruch 4, wobei das Bestimmen der Anzahl von Personen, die diese unterschiedliche Atmungsrate aufweisen (1509), Folgendes umfasst:
für jeden der identifizierten FFT-Peaks:

Filtern der sauberen CSI-Daten, um gefilterte CSI-Daten zu erlangen, die diesem identifizierten FFT-Peak entsprechen;
Bestimmen von Real- und Imaginärteilen der gefilterten CSI-Daten in einer komplexen Ebene;
Detektieren einer Atemphasendifferenz zwischen den Real- und Imaginärteilen; und
Bestimmen der Anzahl von Personen, die diese unterschiedliche Atmungsrate aufweisen, basierend auf der Atemphasendifferenz.

6. Verfahren nach Anspruch 5, wobei das Bestimmen der Anzahl von Personen, die diese unterschiedliche Atmungsrate aufweisen (1509), Folgendes umfasst:
Bestimmen der Anzahl von Personen basierend auf einer kontinuierlichen Reihe von Sofortdetektionsergebnissen der Anzahlen von Personen.

7. Vorrichtung, umfassend:

einen Sendeempfänger, der dazu konfiguriert ist, Kanalzustandsinformationsdaten, CSI-Daten, von Wi-Fi auf einem Sendeantenne/Empfangsantenne-Paar über einen Zeitraum (1501) zu erlangen; und
einen Prozessor, der mit dem Sendeempfänger wirkverbunden ist, wobei der Prozessor zu Folgendem konfiguriert ist:

Entfernen einer oder mehrerer Anomalien, die in den CSI-Daten vorhanden sind (1503);
Durchführen von mindestens einem von Phasenkompensation und Amplitudenkompensation an den CSI-Daten, um saubere CSI-Daten (1505) zu generieren;
Detektieren einer Anzahl von unterschiedlichen Atmungsraten basierend auf den sauberen CSI-Daten (1507); und
für jede der unterschiedlichen Atmungsraten Bestimmen einer Anzahl von Personen, die diese unterschiedliche Atmungsrate aufweisen (1509),
**dadurch gekennzeichnet, dass**
zum Entfernen der einen oder der mehreren Anomalien, die in den CSI-Daten vorhanden sind, der Prozessor zu Folgendem konfiguriert ist:

Sammeln von CSI-Daten für einen vorbestimmten Zeitraum;
Clustern von CSI-Typen innerhalb der während des vorbestimmten Zeitraums gesammelten CSI-Daten unter Verwendung eines Clustering-Algorithmus;
Identifizieren eines normalen CSI-Typs basierend auf den geclusterten CSI-Typen; und
Entfernen eines Teils der CSI-Daten, der nach dem vorbestimmten Zeitraum empfangen wurde und nicht dem normalen CSI-Typ entspricht.

8. Vorrichtung nach Anspruch 7, wobei zum Durchführen der Phasenkompensation an den CSI-Daten (1505) der Prozessor zu Folgendem konfiguriert ist:

Anwenden eines Mittelwertfilters, um einen oder mehrere Phasenfehler aufgrund eines Paketgrenzendetektionsfehlers zu entfernen; und
Subtrahieren einer angepassten linearen Funktion, um einen oder mehrere andere Phasenfehler aufgrund (i) einer Verzögerung, die durch einen Abtastfrequenzversatz verursacht wird, und (ii) eines Frequenzversatzes, der durch einen Mittenfrequenzversatz verursacht wird, zu entfernen.

9. Vorrichtung nach Anspruch 7, wobei zum Durchführen der Amplitudenkompensation an den CSI-Daten (1505) der Prozessor zu Folgendem konfiguriert ist:

Clustern von Amplituden der CSI-Daten unter Verwendung eines Clustering-Algorithmus; und

Normalisieren der CSI-Daten durch Dividieren der CSI-Daten durch eine mittlere CSI-Amplitude eines entsprechenden CSI-Datenclusters.

**10.** Vorrichtung nach Anspruch 7, wobei zum Detektieren der Anzahl von unterschiedlichen Atmungsraten basierend auf den sauberen CSI-Daten (1507) der Prozessor zu Folgendem konfiguriert ist:

Auswählen von Subträgern, die Atmungssignale enthalten, basierend auf Atmungsenergieverhältniswerten, RER-Werten, die aus den sauberen CSI-Daten erlangt werden;

Identifizieren von Peaks einer schnellen Fourier-Transformation, FFT-Peaks, die Atmungsraten verschiedener Personen entsprechen; und

Festlegen der Anzahl von identifizierten FFT-Peaks als die Anzahl von unterschiedlichen Atmungsraten.

**11.** Vorrichtung nach Anspruch 10, wobei zum Bestimmen der Anzahl von Personen, die diese unterschiedliche Atmungsrate aufweisen (1509), der Prozessor zu Folgendem konfiguriert ist:

für jeden der identifizierten FFT-Peaks:

Filtern der sauberen CSI-Daten, um gefilterte CSI-Daten zu erlangen, die diesem identifizierten FFT-Peak entsprechen;

Bestimmen von Real- und Imaginärteilen der gefilterten CSI-Daten in einer komplexen Ebene;

Detektieren einer Atemphasendifferenz zwischen den Real- und Imaginärteilen; und

Bestimmen der Anzahl von Personen, die diese unterschiedliche Atmungsrate aufweisen, basierend auf der Atemphasendifferenz.

**12.** Vorrichtung nach Anspruch 11, wobei der Prozessor zum Bestimmen der Anzahl von Personen, die diese unterschiedliche Atmungsrate aufweisen (1509), basierend auf einer kontinuierlichen Reihe von Sofortdetektionsergebnissen der Anzahlen von Personen konfiguriert ist.

**13.** Nichttransitorisches computerlesbares Medium, umfassend Programmcode, der, wenn er durch einen Prozessor einer Vorrichtung ausgeführt wird, die Vorrichtung zu Folgendem veranlasst:

Erlangen von Kanalzustandsinformationsdaten, CSI-Daten, von Wi-Fi auf einem Sendeantenne/Empfangsantenne-Paar über einen Zeitraum (1501);

Entfernen einer oder mehrerer Anomalien, die in den CSI-Daten vorhanden sind (1503);

Durchführen von mindestens einem von Phasenkompensation und Amplitudenkompensation an den CSI-Daten, um saubere CSI-Daten (1505) zu generieren;

Detektieren einer Anzahl von unterschiedlichen Atmungsraten basierend auf den sauberen CSI-Daten (1507); und

für jede der unterschiedlichen Atmungsraten Bestimmen einer Anzahl von Personen, die diese unterschiedliche Atmungsrate aufweisen (1509),

**dadurch gekennzeichnet, dass**

das Entfernen der einen oder der mehreren Anomalien, die in den CSI-Daten vorhanden sind, Folgendes umfasst:

Sammeln von CSI-Daten für einen vorbestimmten Zeitraum;

Clustern von CSI-Typen innerhalb der während des vorbestimmten Zeitraums gesammelten CSI-Daten unter Verwendung eines Clustering-Algorithmus;

Identifizieren eines normalen CSI-Typs basierend auf den geclusterten CSI-Typen; und

Entfernen eines Teils von CSI-Daten, der nach dem vorbestimmten Zeitraum empfangen wurde und nicht dem normalen CSI-Typ entspricht.

**Revendications**

**1.** Procédé mis en œuvre par ordinateur pour détecter simultanément les fréquences de respiration de plusieurs personnes dans la même pièce, comprenant :

l'obtention de données d'informations d'état de canal Wi-Fi, CSI, sur une paire antenne d'émission/antenne de réception sur une période de temps (1501) ;

la suppression d'une ou plusieurs anomalies présentes dans les données CSI (1503) ;

la réalisation d'au moins l'une d'une compensation de phase et d'uOne compensation d'amplitude sur les données CSI pour générer des données CSI propres (1505) ;

la détection d'un nombre de fréquences de respiration distinctes sur la base des données CSI propres (1507) ; et pour chacune des fréquences de respiration distinctes, la détermination

du nombre de personnes qui comportent cette fréquence

de respiration distinctes (1509),

**caractérisé en ce que**

la suppression de la ou des anomalies présentes dans les données CSI comprend :

la collecte de données CSI pendant une période de temps prédéfinie ;

le regroupement des types de CSI dans les données CSI collectées durant la période de temps prédéfinie à l'aide d'un algorithme de regroupement ;

l'identification d'un type de CSI normal sur la base des types de CSI regroupés ; et

la suppression d'une partie des données CSI reçues après la période de temps prédéfinie qui ne correspond pas au type CSI normal.

2. Procédé de la revendication 1, ladite réalisation de la compensation de phase sur les données CSI (1505) comprenant :

l'application d'un filtre moyen pour supprimer une ou plusieurs erreurs de phase dues à une erreur de détection de limite de paquet ; et

la soustraction d'une fonction linéaire ajustée pour éliminer une ou plusieurs autres erreurs de phase dues à (i) un retard causé par un décalage de fréquence d'échantillonnage et (ii) un décalage de fréquence causé par un décalage de fréquence centrale.

3. Procédé de la revendication 1, ladite réalisation de la compensation d'amplitude sur les données CSI (1505) comprenant :

le regroupement d'amplitudes des données CSI à l'aide d'un algorithme de regroupement ; et

la normalisation des données CSI en divisant les données CSI par une amplitude CSI moyenne d'un groupe de données CSI correspondant.

4. Procédé de la revendication 1, ladite détection du nombre de fréquences de respiration distinctes sur la base des données CSI propres (1507) comprenant :

la sélection de sous-porteuses contenant des signaux de respiration sur la base de valeurs de rapport d'énergie de respiration, RER, obtenues à partir des données CSI propres ;

l'identification des pics de transformée de Fourier rapide, FFT, correspondant aux fréquences de respiration de différentes personnes ; et

la définition du nombre de pics FFT identifiés comme le nombre de fréquences de respiration distinctes.

5. Procédé de la revendication 4, ladite détermination du nombre de personnes qui comportent cette fréquence de respiration distincte (1509) comprenant :

pour chacun des pics FFT identifiés :

le filtrage des données CSI propres pour obtenir des données CSI filtrées correspondant à ce pic FFT identifié ;

la détermination des parties réelles et imaginaires des données CSI filtrées dans un plan complexe ;

la détection d'une différence de phase respiratoire entre les parties réelle et imaginaire ; et

la détermination du nombre de personnes qui comportent cette fréquence de respiration distincte sur la base de la différence de phase de respiration.

6. Procédé de la revendication 5, ladite détermination du nombre de personnes qui comportent cette fréquence de respiration distincte (1509) comprenant :

la détermination du nombre de personnes sur la base d'une série continue de résultats de détection instantanée du nombre de personnes.

**7.** Dispositif comprenant :

un émetteur-récepteur configuré pour recevoir des données d'informations d'état de canal Wi-Fi, CSI, sur une paire antenne d'émission/antenne de réception sur une période de temps (1501) ; et

un processeur connecté fonctionnellement à l'émetteur-récepteur, le processeur étant configuré pour :

supprimer une ou plusieurs anomalies présentes dans les données CSI (1503) ;
réaliser au moins l'une d'une compensation de phase et d'une compensation d'amplitude sur les données CSI pour générer des données CSI propres (1505) ;
détecter un nombre de fréquences de respiration distinctes sur la base des données CSI propres (1507) ; et
pour chacune des fréquences de respiration distinctes, déterminer un nombre de personnes qui comportent cette fréquence de respiration distincte (1509),
**caractérisé en ce que**
pour supprimer la ou les anomalies présentes dans les données CSI, le processeur est configuré pour :

collecter des données CSI pendant une période de temps prédéfinie ;
regrouper des types de CSI dans les données CSI collectées durant la période de temps prédéfinie à l'aide d'un algorithme de regroupement ;
identifier un type de CSI normal sur la base des types de CSI regroupés ; et
supprimer une partie des données CSI reçues après la période de temps prédéfinie qui ne correspond pas au type CSI normal.

**8.** Dispositif de la revendication 7, pour réaliser la compensation de phase sur les données CSI (1505), ledit processeur étant configuré pour :

appliquer un filtre moyen pour supprimer une ou plusieurs erreurs de phase dues à une erreur de détection de limite de paquet ; et
soustraire une fonction linéaire ajustée pour éliminer une ou plusieurs autres erreurs de phase dues à (i) un retard causé par un décalage de fréquence d'échantillonnage et (ii) un décalage de fréquence causé par un décalage de fréquence centrale.

**9.** Dispositif de la revendication 7, pour réaliser la compensation d'amplitude sur les données CSI (1505), ledit processeur étant configuré pour :
regrouper des amplitudes des données CSI à l'aide d'un algorithme de regroupement ; et normaliser les données CSI en divisant les données CSI par une amplitude CSI moyenne d'un groupe de données CSI correspondant.

**10.** Dispositif de la revendication 7, pour détecter le nombre de fréquences de respiration distinctes sur la base des données CSI propres (1507), ledit processeur étant configuré pour :

sélectionner des sous-porteuses contenant des signaux de respiration sur la base de valeurs de rapport d'énergie de respiration, RER, obtenues à partir des données CSI propres ;
identifier des pics de transformée de Fourier rapide, FFT, correspondant aux fréquences de respiration de différentes personnes ; et
définir le nombre de pics FFT identifiés comme le nombre de fréquences de respiration distinctes.

**11.** Dispositif de la revendication 10, pour déterminer le nombre de personnes qui comportent cette fréquence de respiration distincte (1509), ledit processeur étant configuré pour :
pour chacun des pics FFT identifiés :

filtrer les données CSI propres pour obtenir des données CSI filtrées correspondant à ce pic FFT identifié ;
déterminer des parties réelles et imaginaires des données CSI filtrées dans un plan complexe ;
détecter une différence de phase de respiration entre les parties réelle et imaginaire ; et
déterminer le nombre de personnes qui comportent cette fréquence de respiration distincte sur la base de la différence de phase de respiration.

**12.** Dispositif de la revendication 11, ledit processeur étant configuré pour déterminer le nombre de personnes qui comportent cette fréquence de respiration distincte (1509) sur la base d'une série continue de résultats de détection instantanée du nombre de personnes.

13. Support non transitoire lisible par ordinateur comprenant un code de programme qui, lorsqu'il est exécuté par un processeur d'un dispositif, amène le dispositif à :

obtenir des données d'informations d'état de canal Wi-Fi, CSI, sur une paire antenne d'émission/antenne de réception
sur une période de temps (1501) ;
supprimer une ou plusieurs anomalies présentes dans les données CSI (1503) ;
réaliser au moins l'une d'une compensation de phase et d'une compensation d'amplitude sur les données CSI pour générer des données CSI propres (1505) ;
détecter un nombre de fréquences de respiration distinctes sur la base des données CSI propres (1507) ; et
pour chacune des fréquences de respiration distinctes, déterminer un nombre de personnes qui comportent cette fréquence de respiration distincte (1509),
**caractérisé en ce que**
la suppression de la ou des anomalies présentes dans les données CSI comprend :

la collecte de données CSI pendant une période de temps prédéfinie ;
le regroupement des types de CSI dans les données CSI collectées durant la période de temps prédéfinie à l'aide d'un algorithme de regroupement ;
l'identification d'un type de CSI normal sur la base des types de CSI regroupés ; et
la suppression d'une partie des données CSI reçues après la période de temps prédéfinie qui ne correspond pas au type CSI normal.

[Fig. 1]

[Fig. 2a]

[Fig. 2b]

[Fig. 3]

300

301
CSI Data

310
Motion Detection

320
Anomaly Removal

330
CSI Amplitude/Phase Compensation

332
Clean CSI

340
Respiration rate detection

EP 4 358 837 B1

[Fig. 4]

320

301
CSI
Data

First 30
sec?
410

True → Collect 30 sec CSI Data (420) → Cluster CSI types using DBSCAN (430) → Identify the normal CSI type (440)

Cluster centroids

False → Normal CSI type? (450)

True → Keep this CSI (460)

False → Remove this CSI (470)

[Fig. 5]

330

301 Raw CSI

510 phase

520 amplitude

512 Apply mean filter to remove phase errors due to PBD

522 Utilize DBSCAN to cluster CSI amplitudes

514 Subtract by linear fitting function to remove phase errors due to SFO and CFO

524 Normalize CSI amplitude with cluster mean

phase

amplitude

332 Clean CSI $H_k(t)$

[Fig. 6]

EP 4 358 837 B1

510

**601** The noisy CSI phase $\phi_k(t)$ is modeled as:
$$\phi_k(t) = \theta_k(t) + 2\pi\Delta f \cdot (\tau_{\mathrm{PBD}}(t) + \tau_{\mathrm{SFO}}(t) + \tau_{\mathrm{ToF}}(t)) \cdot k + \beta_{\mathrm{CFO}}(t).$$

**603** For each subcarrier $k$, calculate the mean value of $\phi_k(t)$ to remove $\tau_{\mathrm{PBD}}(t)$ since $\tau_{\mathrm{PBD}}(t)$ follows zero-mean Gaussian distribution.
$$\bar{\phi}_k(t) \approx \bar{\theta}_k(t) + 2\pi\Delta f \cdot (\bar{\tau}_{\mathrm{SFO}}(t) + \bar{\tau}_{\mathrm{ToF}}(t)) \cdot k + \bar{\beta}_{\mathrm{CFO}}(t).$$

**605** At each time t, apply a least square linear fitting on $\bar{\phi}_k(t)$ to estimate the slope and bias:
$$\hat{\alpha}(t) \approx 2\pi\Delta f \cdot (\bar{\tau}_{\mathrm{SFO}}(t) + \bar{\tau}_{\mathrm{ToF}}(t)), \quad \hat{\beta}(t) \approx \bar{\beta}_{\mathrm{CFO}}(t)$$

**607** The CSI phase is calculated by $\hat{\theta}_k(t) = \bar{\phi}_k(t) - \hat{\alpha}(t) \cdot k - \hat{\beta}(t)$

[Fig. 7]

[Fig. 8]

EP 4 358 837 B1

340

332
Clean CSI

805
Calculate RER for each subcarrier

810
Select subcarrier with top x% RER

815
FFT of selected CSI

820
FFT Peak detection

825
$N$ peaks on $f_1, ..., f_N$
Instant # People $\leftarrow N$
$i \leftarrow 1$

830
$i \leq N$
True / False

835
For $f_i$, diff btw Real and Imaginary breathing phase > threshold
True / False

840
Instant # People $\leftarrow$ Instant # People $+ 1$

845
$i \leftarrow i + 1$

850
Instant # People

855
Majority vote to determine # People

860
Respiration rate Matching

865
Output:
1. # People
2. Their respiration rates

[Fig. 9]

805

332 — CSI of each subcarrier

910 — Interpolation and apply FFT within $T$ time window

920 — Detect the frequency $f_{max}$ (bpm) within the maximum FFT peak in the range of $[r_1, r_2]$ bpm

930 — $RER = \dfrac{\textit{Energy of FFT within } [f_{max}-2, f_{max}+2] \textit{ bpm}}{\textit{Energy of all FFT}}$

[Fig. 10]

[Fig. 11]

820

```
┌─────────────────────────────────┐
│     Selected CSI subcarriers     │
└─────────────────────────────────┘
                 │
                 ▼
┌─────────────────────────────────┐
│   Interpolation and apply FFT    │
│     within T time window         │
└─────────────────────────────────┘
                 │
                 ▼
┌─────────────────────────────────┐
│ Select FFT peaks f¹, f² ... with y% │
│         largest amplitude        │
└─────────────────────────────────┘
                 │
                 ▼
┌─────────────────────────────────┐
│ Select FFT peak fᵢ with the largest │
│             amplitude            │
└─────────────────────────────────┘
                 │
                 ▼
┌─────────────────────────────────┐
│ Remove FFT peaks that |f − fᵢ| < │
│             1/T bpm              │
└─────────────────────────────────┘
```

$i \leftarrow i + 1$

Select FFT peak $f_i$ with the largest amplitude

Remove FFT peaks that $|f - f_i| < 1/T$ bpm

Remaining FFT peaks?

True

False

The number of distinct respiration rates

$f_1, f_2, \ldots$

[Fig. 12a]

1201

[Fig. 12b]

1202

[Fig. 13]

32

1300

1305
Obtain real and imaginary parts of CSI $\mathcal{H}_k(t)$

$\mathrm{Re}\{\mathcal{H}_k(t)\}$

$\mathrm{Im}\{\mathcal{H}_k(t)\}$

1310
Detect the breathing phase difference $\varphi_k^{\mathrm{diff}}$

$\varphi_k^{\mathrm{diff}}$

1315
Feature extraction

1320
Classifier

Number of people

[Fig. 14]

EP 4 358 837 B1

860

| Detected respiration rates at time $t$, $f_1^t, \ldots f_N^t$ | → | $i \leftarrow 1$ | → | Match $f_i^t$ with $f_j^{t-1}$ such that $j = \min_k |f_i^t - f_k^{t-1}|$ | → | $i \leftarrow i + 1$ | → | $i \leq N$ |

True

False

$t \leftarrow t + 1$

[Fig. 15]

1500

```
                           ┌─────────────┐
                           │    START    │
                           └──────┬──────┘
                                  │
                                  ▼
      ┌──────────────────────────────────────────────────────────┐
1501  │  OBTAIN WI-FI CSI DATA ON A TRANSMIT ANTENNA/RECEIVE       │
      │          ANTENNA PAIR OVER A TIME PERIOD                   │
      └──────────────────────────┬───────────────────────────────┘
                                 │
                                 ▼
      ┌──────────────────────────────────────────────────────────┐
1503  │  REMOVE ONE OR MORE ANOMALIES PRESENT IN THE CSI          │
      │                      DATA                                  │
      └──────────────────────────┬───────────────────────────────┘
                                 │
                                 ▼
      ┌──────────────────────────────────────────────────────────┐
1505  │  PERFORM PHASE AND/OR AMPLITUDE COMPENSATION ON           │
      │      THE CSI DATA TO GENERATE CLEAN CSI DATA               │
      └──────────────────────────┬───────────────────────────────┘
                                 │
                                 ▼
      ┌──────────────────────────────────────────────────────────┐
1507  │  DETECT A NUMBER OF DISTINCT RESPIRATION RATES BASED      │
      │              ON THE CLEAN CSI DATA                         │
      └──────────────────────────┬───────────────────────────────┘
                                 │
                                 ▼
      ┌──────────────────────────────────────────────────────────┐
1509  │  FOR EACH OF THE DISTINCT RESPIRATION RATES,              │
      │  DETERMINE A NUMBER OF PEOPLE THAT HAVE THAT              │
      │           DISTINCT RESPIRATION RATE                        │
      └──────────────────────────┬───────────────────────────────┘
                                 │
                                 ▼
                           ┌─────────────┐
                           │    STOP     │
                           └─────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017156492 A1

- US 10735298 B2

**Non-patent literature cited in the description**

- **WANG XUYU et al.** PhaseBeat: Exploiting CSI Phase Data for Vital Sign Monitoring with Commodity WiFi Devices. *Proceedings of the International Conference on Distributed Computing Systems*, 05 June 2017, 1230-1239

- **ZHANG FUSANG et al.** Unlocking the Beamforming Potential of LoRa for Long-range Multi-target Respiration Sensing. *Proceedings of the ACM on Interactive, Mobile, Wearable and Ubiquitous Technologies*, 24 June 2021, vol. 5 (2), 1-25